# EUROPEAN PATENT SPECIFICATION

(11) **EP 2 670 289 B1**
(45) Date of publication and mention of the grant of the patent: **10.01.2018**
(21) Application number: 12702731.6
(22) Date of filing: 27.01.2012
(51) Int. Cl.: A61B 1/005, A61B 1/008

(54) **ARTICULATION JOINTS FOR TORQUE TRANSMISSION**
GELENKVERBINDUNGEN ZUR DREHMOMENTÜBERTRAGUNG
JONCTIONS D'ARTICULATION POUR LA TRANSMISSION DE COUPLE

(30) Priority: 31.01.2011 US 201161438072 P
(43) Date of publication of application: 11.12.2013
(73) Proprietor: Boston Scientific Scimed, Inc., Maple Grove, MN 55311-1566 (US)
(72) Inventor: GOLDEN, John, Norton, MA 02766 (US); SMITH, Paul, Smithfield, RI 02917 (US); BARNER, Paul, Arlington, MA 02474 (US); CAMPBELL, Andrew, Reading, MA 01867 (US); KAPPEL, Gary, Acton, MA 01720 (US)
(74) Representative: Vossius & Partner Patentanwälte Rechtsanwälte mbB
(86) International application number: PCT/US2012/022835
(87) International publication number: WO 2012/106186

(56) References cited:
- WO-A2-2009/005274
- US-A- 5 448 989
- US-A1- 2006 199 999
- US-A1- 2011 009 863

## Description

This application claims the benefit of priority from U.S. Provisional Application No. 61/438,072, filed January 31, 2011.

### Field of the Invention

Embodiments of the present invention relate to articulation joints for use with medical devices. In particular, embodiments of the present invention include articulation links that provide torque transmission.

### Background of the Invention

Articulated medical devices provide access to sites within a patient's body that are difficult to reach using rigid non-articulating devices. For example, articulated endoscopes provide direct access to internal organs, and articulated catheters provide access to tortuous vascular structures. Examples of articulated medical devices are disclosed in patent documents US2011009863A1, WO2009005274A2, US2006199999A1 and US5448989A.

Articulated medical devices have traditionally been flexible to provide easy manipulation. An instrument must have sufficient flexibility to navigate through the lower gastro-intestinal tract. Increasing the articulation joints¹ flexibility reduces the forces required to articulate the device, reducing the device's load bearing requirements and operator fatigue.

However, traditional articulation joints often lack the ability to efficiently transfer torque along the length of the medical device. Highly flexible articulated joints often contain significant torsional lag and elasticity. Applied torsional forces are often absorbed by torsional movement between adjacent articulation joints. Multiple articulation joints cumulate these torsional losses, reducing the efficiency of torsional forces transmitted along the length of the device.

The articulating links described herein overcome these and other limitations of the prior art. The articulation links of the present disclosure are broadly applicable to various medical devices and other devices requiring articulation. For example, borescopes use articulation to access difficult-to-reach locations within engines or other industrial devices.

### SUMMARY OF THE INVENTION

According to the present invention an articulation link includes a body defining a longitudinal axis and a transverse plane extending perpendicular to the longitudinal axis, the body having a first surface located proximate a first end of the body and a second surface located proximate a second end of the body opposite the first end. A cavity extends from the first surface to a cavity contact surface located between the first surface and the second surface, wherein the cavity contact surface extends parallel to the transverse plane. The articulation link also includes a protrusion extending from the second surface away from the first surface to a protrusion contact surface, wherein the protrusion contact surface is curved and configured to engage a cavity contact surface of an adjacent link, and wherein the cavity includes a cavity sidewall extending perpendicular to the transverse plane, and the protrusion includes a protrusion sidewall configured to engage a cavity sidewall of the adjacent link.

The articulation link includes a body defining a longitudinal axis and a transverse plane extending perpendicular to the longitudinal axis, the body having a first surface located proximate a first end of the body and a second surface located proximate a second end of the body opposite the first end. The articulation link also includes a cavity extending from the first surface to a cavity contact surface located between the first surface and the second surface. Also, a protrusion extends from the second surface, wherein the protrusion includes at least two curved rails configured to engage a cavity contact surface of an adjacent link, and wherein the cavity includes a cavity sidewall extending perpendicular to the transverse plane, and the protrusion includes a protrusion sidewall extending parallel to the cavity sidewall. The articulation link also includes a lumen extending longitudinally through the body.

In another aspect, a medical instrument can include an elongate member configured to actuate the medical instrument, and a plurality of articulation links. Each articulation link can include a body defining a longitudinal axis and a transverse plane extending substantially perpendicular to the longitudinal axis, the body having a first surface located proximate to a first end of the body, a second surface located proximate to a second end of the body opposite the first end, and a lumen configured to receive the elongate member. Each link can also include a cavity extending from the first surface to a cavity contact surface located between the first surface and the second surface, wherein the cavity contact surface can extend parallel to the transverse plane. A protrusion may extend from the second surface away from the first surface to a protrusion contact surface, wherein the protrusion contact surface can be curved and configured to engage the cavity contact surface of an adjacent link, and wherein the cavity can include a cavity sidewall extending substantially perpendicular to the transverse plane, and the protrusion can include a protrusion sidewall parallel to a cavity sidewall of the adjacent link. Each articulation link can include a central lumen extending longitudinally from the cavity contact surface to the protrusion contact surface.

Additional objects and advantages of the invention will be set forth in part in the description which follows, and in part will be obvious from the description, or may be learned by practice of the invention. The objects and advantages of the invention will be realized and attained by means of the elements and combinations particularly pointed out below.

### BRIEF DESCRIPTION OF THE DRAWINGS

The accompanying drawings, which are incorporated in and constitute a part of this specification and together with the description, serve to explain the principles of the invention.
FIG. 1 is a perspective view of a device, according to an exemplary embodiment of the invention;
FIG. 2A is perspective view of the top of an articulation link, according to the invention;
FIG. 2B is perspective view of the bottom of an articulation link, according the invention;
FIG. 3A is a top view of an articulation link, according to an exemplary embodiment of the invention;
FIG. 3B is a bottom view of an articulation link, according to an exemplary embodiment of the invention;
FIG. 4 is a cut-away side view of the articulation link as shown in FIG. 3A, according to an exemplary embodiment of the invention;
FIG. 5A is a side-view of an articulation link, according to an exemplary embodiment of the invention;
FIG. 5B is a side-view of an articulation link, according to an exemplary embodiment of the invention;
FIG. 5C is a side-view of an articulation link, according to an exemplary embodiment of the invention;
FIG. 6A is perspective view of the top of an articulation link, according to another exemplary embodiment of the invention;
FIG. 6B is perspective view of the bottom of an articulation link, according to another exemplary embodiment of the invention;
FIG. 7 is a perspective view of a plurality of articulation links, according to an exemplary embodiment of the invention; and
FIG. 8 is a cut-away side view of a plurality of articulation links, according to an exemplary embodiment of the invention.

### DESCRIPTION OF THE EMBODIMENTS

Reference will now be made to the invention and to exemplary embodiments of the invention, examples of which are illustrated in the accompanying drawings. Wherever possible, the same reference numbers will be used throughout the drawings to refer to the same or like parts.

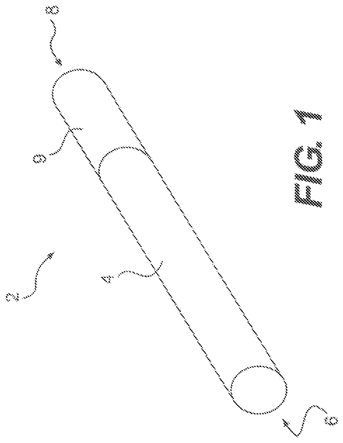

FIG. 1 depicts a device 2, according to an exemplary embodiment. Device 2 can include a medical device configured for use with a surgical method, including a therapeutic or diagnostic procedure. Device 2 can include an endoscope, a guide tube, a catheter, a guidewire, or other type of elongate medical device. For example, device 2 may be used for procedures within or adjacent to various body organs, such as, an esophagus, a heart, a stomach, a pelvic area, a bladder, an intestine, or a gastrointestinal, urinary, or pulmonary tract.

Device 2 may be configured for insertion into a patient's body through an anatomical opening. In other embodiments, device 2 may be used in natural orifice transluminal endoscopic surgery (NOTES) procedures or single incision laparoscopic surgical (SILS) procedures. Accordingly, device 2 can be shaped and sized for placement into a patient via a body cavity or an incision.

Device 2 can be configured to operate with one or more instruments (not shown) used in various surgical procedures. For example, device 2 can include one or more lumens (not shown) configured to receive a grasper, a pair of scissors, a hook, an ablation device, or other type of surgical instrument. Device 2 may also be configured to operate with, or include, insufflation, irrigation, suction, imaging, or systems used in endoscopic, laparoscopic, or other surgical procedures.

Device 2 can include a shaft 4 having a proximal end 6 and a distal end 8. Proximal end 6 can be manipulated by an operator to control distal end 8. For example, proximal end 6 can include one or more knobs, handles, or other devices (not shown) configured to move distal end 8 relative to proximal end 6.

Shaft 4 can also include an articulating portion 9. As shown in FIG. 1, articulating portion 9 can be located at or near distal end 8. In other embodiments, articulating portion 9 can be located anywhere along shaft 4, or encompass the entire length of shaft 4. In operation, an operator can manipulate articulation portion 9 to move distal end 8 up, down, left, or right.

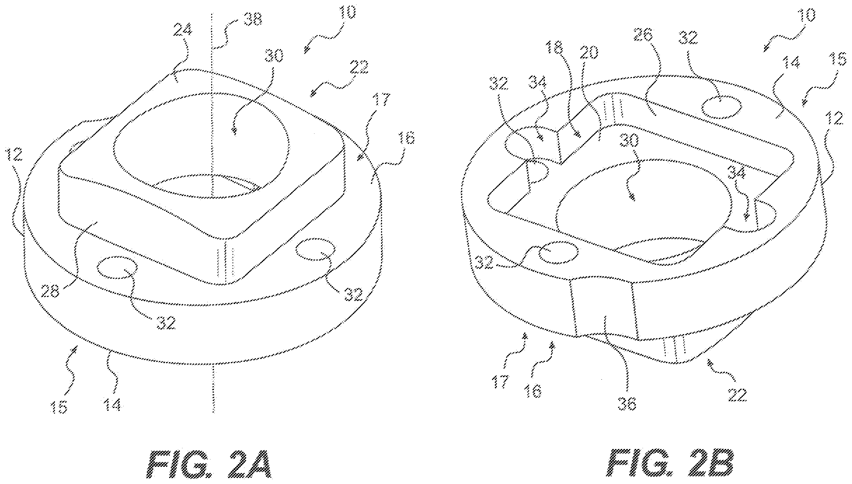

FIGS. 2A and 2B depict an articulation link 10, according to the invention Articulation link 10 can be configured for use with medical device 2. For example, a plurality of articulation links 10 can be used to form part of articulation portion 9, as shown in FIG. 1.

Articulation link 10 includes a body 12. Body 12 has a cylindrical shape and a circular cross-section. In other embodiments, body 12 can have a different shape or cross-section, and may be shaped and sized as required.

Body 12 can be formed from one or more materials, such as, for example, a metal alloy, a polymer, a ceramic, or combination thereof. One or more external surfaces of body 12 may be treated to enhance lubricity, friction, hardness, strength, or another physical parameter. All or part of body 12 may be coated with various materials to improve these and other physical parameters.

Body 12 includes a first surface 14 and a second surface 16. First surface 14 is located proximally and second surface 16 is located distally. Body 12 has first surface 14 located proximate a first end 15 of body 12 and second surface 16 located proximate a second end 17 of body 12 opposite first end 15. In some instances, first surface 14 can be the proximal-most surface and second surface 16 can be the distal-most surface of body 12. In other embodiments, first surface 14 can be distal of second surface 16 or the distal-most surface of body 12.

As shown in FIGS. 2A and 2B, surfaces 14, 16 can be flat. In other embodiments, surfaces 14, 16 can be curved, irregular, or include another type of surface.

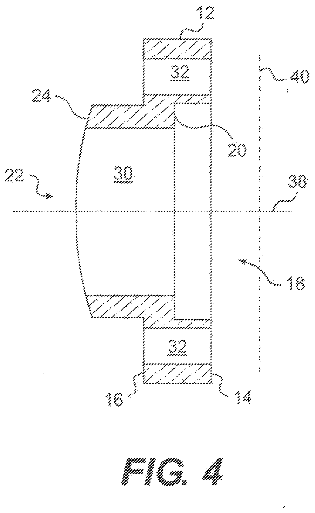

Body 12 includes a cavity 18 located within body 12 and extending distally from first surface 14. As shown in FIGS. 2B and 4, cavity 18 includes a cavity contact surface 20 located between first surface 14 and second surface 16. Cavity contact surface 20 can be flat, curved, or shaped as required. Cavity 18 includes one or more cavity sidewalls 26. Cavity sidewall 26 is flat.

While cavity 18 is shown and described herein as generally rectangular, cavity 18 can include any suitable shape. For example, cavity 18 could have a non-rectangular geometry. In other embodiments, cavity 18 could include any number of sidewalls 26. Sidewalls 26 could be elliptical or non-symmetrical, and may include various slots or other shapes extending into or out of sidewall 26. Cavity 18 could include multiple teeth or tabs to form a star or a gear shape.

Body 1 includes a protrusion 22 configured to engage a cavity 18 of an adjacent link 10. As explained below, cavity 18 and protrusion 22 are configured to transfer torque between adjacent articulation links 10.

As shown in FIG. 2A, protrusion 22 extends distally from second surface 16. Protrusion 22 includes a protrusion contact surface 24 located distally from body 12. Protrusion contact surface 24 is curved in one or more directions, as explained below. Further, protrusion contact surface 24 is configured to engage cavity contact surface 20. In some instances, protrusion contact surface 24 can be the distal-most surface of protrusion 22.

Protrusion 22 includes one or more protrusion sidewalls 28. Protrusion sidewall 28 is configured to engage cavity sidewall 26 of an adjacent link 10 to permit the transfer of torque between adjacent articulation links 10. In a further aspect, two or more cavity sidewalls 26 are parallel or two or more protrusion sidewalls 28 are parallel. Protrusion sidewall 28 is flat.

Torsional forces between adjacent articulation links 10 can be transferred from protrusion 22 of one link 10 to cavity 18 of an adjacent link 10. As explained below, torsional forces can be transferred from protrusion sidewall 28 of one link 10 to cavity sidewall 26 of an adjacent link 10. As articulation link 10 is rotated about a longitudinal axis 38, protrusion sidewall 28 engages cavity sidewall 26 of a distally located adjacent articulation link 10. Engagement between sidewalls 26, 28 permits torque transfer while retaining sufficient movement between adjacent articulation links 10.

Body 12 also includes a lumen 30. Lumen 30 extends through body 12, including its protrusion 22. As shown in FIGS. 2A and 2B, lumen 30 can extend from cavity contact surface 20 to protrusion contact surface 24. Lumen 30 can provide communication with cavity 18 such that a medical device may pass through body 12. For example, lumen 30 can be configured to receive one or more elongate medical instruments (not shown). Lumen 30 can also be shaped and sized as required. Further, lumen 30, cavity 18, or protrusion 22 are centered on longitudinal axis 38.

Body 12 further includes one or more lumens 32 configured to receive a cable, wire, or other type of elongate member (not shown) for articulation of one or more articulation links 10. Lumens 32 include between first surface 14 and second surface 16 or between cavity contact surface 20 and second surface 16. Body 12 also includes one or more recesses 34 each configured to receive an elongate member passing through cavity contact surface 20.

In some instances, body 12 can include an alignment marker 36. As shown in FIG. 2B, alignment marker 36 can be located in a sidewall of body 12. In other embodiments, alignment marker 36 can be located on another part of body 12 or its protrusion 22. Alignment marker 36 can provide a visual marker to assist alignment of adjacent articulation links 10. Alignment marker 36 can include a pimple, divot, or other type of physical feature. In other embodiments, alignment marker 36 can include a line, a spot, or other visual feature.

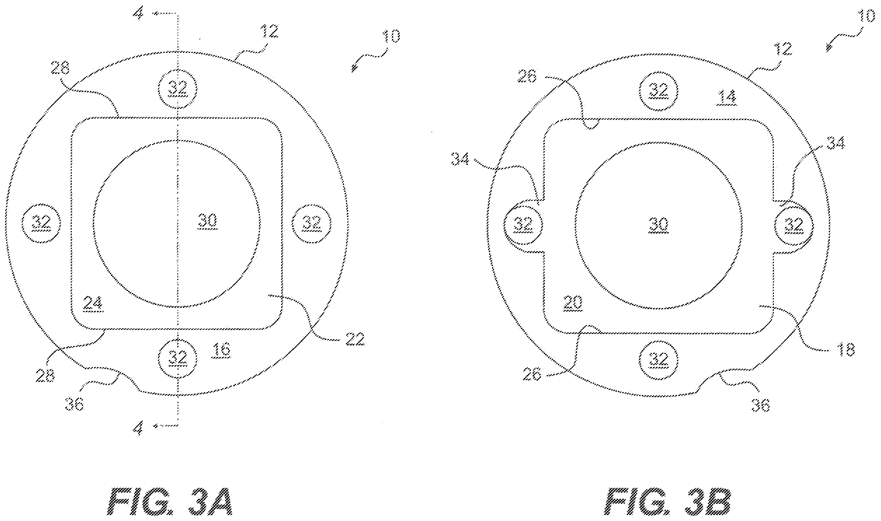

FIGS. 3A and 3B are top and bottom views, respectively, of body 12 showing an arrangement of features transverse to the longitudinal axis, according to an exemplary embodiment. FIG. 3A shows a top view of the distal facing surfaces of body 12, including second surface 16 and protrusion contact surface 24. Protrusion 22 is shown as having a generally square transverse cross-section.

FIG. 3B shows a bottom view of the proximal facing surfaces of body 12, including first surface 14 and cavity contact surface 20. Cavity 18 is shown as having a generally rectangular transverse cross-section. Although cavity 18 and protrusion 22 are shown in FIGS. 3A and 3B as quadrilateral, the transverse cross-sections of cavity 18 and protrusion 22 can be any shape capable of transmitting torque between adjacent articulation links 10. For example, a transverse cross-section of cavity 18 or protrusion 22 can include a slot, a triangle, or a polygon. Cavity 18 or protrusion 22 can also include a curved transverse cross-section.

FIG. 4. shows a cut-away side view of body 12 with longitudinal axis 38 (dotted line) extending therethrough and a transverse plane 40 (dashed line) that extends substantially perpendicular to longitudinal axis 38. As shown in FIG. 4, cavity contact surface 20 can extend parallel to transverse plane 40. First surface 14 or second surface 16 can also be parallel to transverse plane 40.

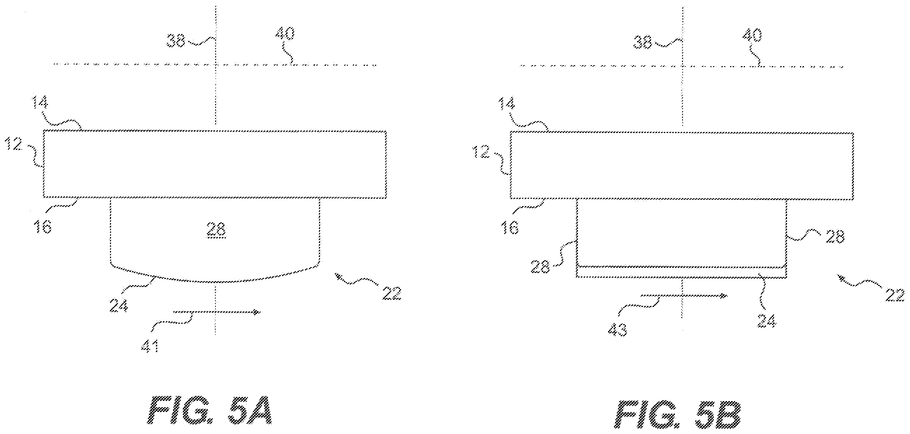

FIG 5A and 5B show main body 12. The view of body 12 in FIG. 5B is rotated 90 degrees about axis 38 relative to the view in FIG. 5A. FIG. 5A illustrates protrusion contact surface 24 being generally curved. In some embodiments, protrusion contact surface 24 can be generally semi-cylindrical in shape. That is, protrusion contact surface 24 can be generally convex in a first direction 41, as shown in FIG. 5A, with substantially straight side edges in a second direction 43, as shown in FIG. 5B. Directions 41, 43 both lie parallel to transverse plane 40 and both directions 41, 43 are perpendicular to each other, as shown in FIGS. 5A and 5B.

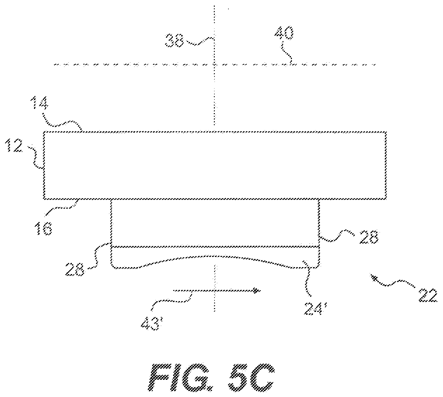

In another embodiment, protrusion contact surface 24 can be curved in first direction 41, as shown in FIG. 5A, and have a non-linear shape in another direction. For example, as shown in FIG. 5C, protrusion contact surface 24' can be generally concave in second direction 43', wherein directions 41 and 43' are perpendicular to each other.

Protrusion contact surface 24 can be variously shaped to permit protrusion 22 of a proximal articulation link 10 to slide relative to cavity 18 of an adjacent distal articulation link 10. Such sliding can occur in one plane, such as, for example, in transverse plane 40. In other embodiments, sliding can occur in one direction, such as, for example, in direction 41. Similarly, adjacent articulation links 10 may also rotate in relation to each other about one or more planes or axes.

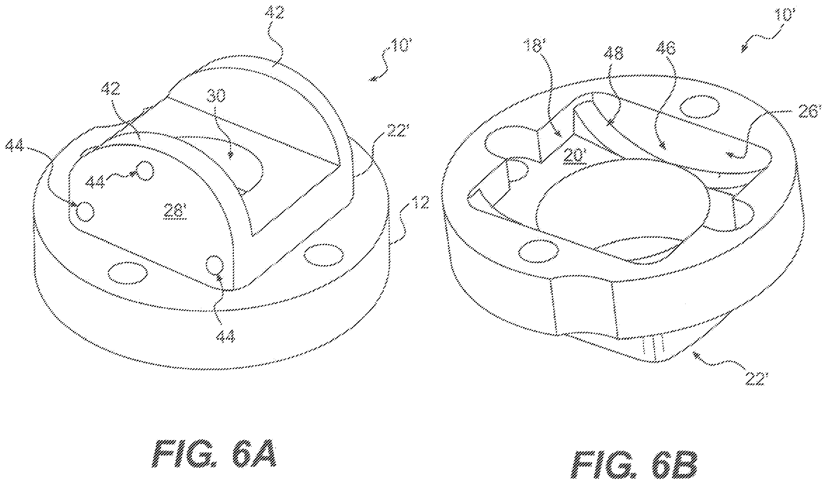

FIGS. 6A and 6B illustrate another embodiment of articulation link 10', wherein protrusion 22' includes one or more rails 42. Two rails 42 are shown as generally curved and are configured to engage cavity contact surface 20' of an adjacent articulation link 10', as shown in FIG. 6B. Cavity contact surface 20' can include one or more recesses 46 configured to receive one or more rails 42.

Protrusion sidewall 28' can include one or more contact features 44, as shown in FIG. 6A. Contact feature 44 can be configured to provide retaining forces between adjacent links 10' to assist maintaining the relative positioning of adjacent links 10'. For example, contact feature 44 of articulation link 10' can be configured to fit within a groove 48 configured to receive contact feature 44 of adjacent link 10', as shown in FIG. 6B.

Contact feature 44 could include a pimple, a ridge, an indentation, a divot, or similar structure. Contact feature 44 could also be configured to at least partially deform. Such a "sacrificial" feature may partially deform to provide a tight fit between contact feature 44 and groove 48 of an adjacent link 10'. Groove 48 can include any appropriate structure configured to engage feature 44.

Cavity sidewall 26' can include one or more grooves 48 configured to receive contact features 44. Protrusion sidewall 28', cavity sidewall 26', recess 46, and rail 42 may include one or more contact features 44 or grooves 48 to facilitate the attachment of two adjacent articulation links 10'.

It is also contemplated that protrusion 22 and cavity 18, as shown in FIGS. 2A and 2B, could also include one or more features 44 or grooves 48. Contact feature 44 and groove 48 can permit the relative movement between sidewalls 26, 28, while maintaining torque transfer between adjacent articulation links 10.

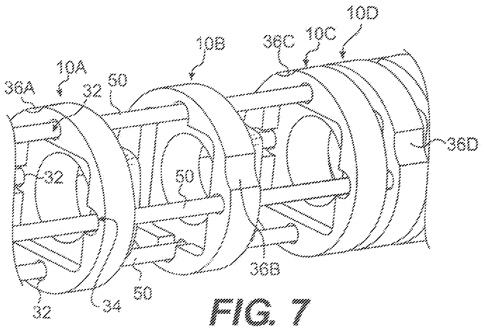

FIG. 7 depicts a partially exploded view of a plurality of articulation links 10A-10D. Articulation links 10A-10D are shown as alternating in their transverse alignment, with alignment markers 36A-36D shown as alternating relative to adjacent markers. As shown in FIG. 7, articulation links 10A-10D are rotated 90 degrees relative to each other. Also four cables 50 are shown passing through four cable lumens 32.

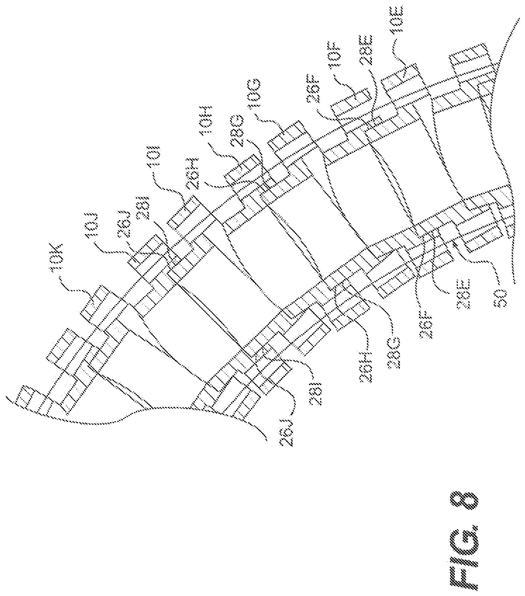

In operation, articulation links 10A-10D would be closely stacked, as shown between articulation links 10C and 10D. A distal region of cable 50 can be restrained relative to an articulation link. Pulling one cable 50 proximally (i.e., downwards, as shown in FIG. 8) can articulate the plurality of articulation links. Articulation links 10A-10D can be directed in up/down or left/right directions due to the alternating stacking of articulation links 10A-10D. That is, articulation links 10A and 10B can rotate in a first direction (e.g., up/down) and articulation links 10B and 10C can rotate in a second direction that is perpendicular to the first direction (e.g., left/right). Such alternating stacking of articulation links 10A-10C permits the stacked articulation links to be articulated in up/down and left/right directions. In other embodiments, articulation links 10 may articulate in only one direction (i.e., up/down or left/right).

FIG. 8 shows a cut-away side view of a plurality of articulation links 10E-10K curving to the left. When tension is applied to cable 50, select adjacent articulation links 10E-10K rotate relative to one another. As shown in FIG. 8, articulation links 10F, 10H, and 10J rotate with respect to their adjacent articulation links located above (i.e., articulation links 10G, 101, and 10K, respectively), but do not rotate with respect to their adjacent articulation links located below (i.e., articulation links 10E, 10G, and 101, respectively). That is, articulation links 10E and 10F, 10G and 10H, and 101 and 10J remain parallel to each other. Moreover, protrusion sidewalls 28E, 28G, and 28I remain parallel to and engaged with cavity sidewalls 26F, 26H, and 26J, respectively. Because these sidewalls remain engaged over a range of articulation angles, torsional forces are efficiently transferred between adjacent articulation links 10E-10K. Providing high tolerances between these sidewalls can further reduce torsional losses. Further, pulling a cable located 90 degrees from cable 50 (in a transverse plane) can result in other pairs of adjacent links 10E-10K rotating relative to each other so that the chain of articulation links 10 rotates into or out of the page as indicated in FIG. 8.

Other embodiments of the invention will be apparent to those skilled in the art from consideration of the specification and practice of the invention disclosed herein. It is intended that the specification and examples be considered as exemplary only, the invention being indicated by the following claims.

## Claims

1. An articulation link, comprising:
a body (12) defining a longitudinal axis (38) and a transverse plane extending substantially perpendicular to the longitudinal axis, the body having a first surface (14) located proximate a first end (15) of the body and a second surface (16) located proximate a second end (17) of the body opposite the first end;
a cavity (18) extending from the first surface (14) to a cavity contact surface (20) located between the first surface and the second surface, wherein the cavity contact surface extends parallel to the transverse plane; and
a protrusion (22) extending from the second surface (16) away from the first surface to a protrusion contact surface (24), wherein the protrusion contact surface is curved and configured to engage a cavity contact surface of an adjacent link,
wherein the cavity includes a cavity sidewall (26) extending perpendicular to the transverse plane, and the protrusion (22) includes a protrusion sidewall (28) configured to engage the cavity sidewall (26) of the adjacent link, and
wherein the protrusion (22) is centered on the longitudinal axis.

2. The articulation link of claim 1, wherein the cavity sidewall (26) of the articulation link (10) is parallel to the protrusion sidewall (28).

3. The articulation link of claim 2, wherein the cavity sidewall (26) of the articulation link (10) and the protrusion sidewall (28) of the adjacent link are flat.

4. The articulation link of claim 3, wherein at least one of the first surface and the second surface (14, 16) is flat and parallel to the transverse plane.

5. The articulation link of claim 2, further including at least two parallel cavity sidewalls (26) and at least two parallel protrusion sidewalls (28).

6. The articulation link of claim 5, wherein each transverse cross-section of the cavity and the protrusion is rectangular.

7. The articulation link of claim 1, wherein the protrusion contact surface (24) is semi-cylindrical.

8. The articulation link of claim 1, wherein the protrusion contact surface (24) is convex in a first direction in the transverse plane and concave in a second direction in the transverse plane, the first direction being perpendicular to the second direction.

9. The articulation link of claim 1, wherein the body (12) includes a central lumen (30) extending longitudinally from the cavity contact surface (20) of the articulation link to the protrusion contact surface (24), and the body further includes at least one lumen (32) extending between the first surface (14) and the second surface (16).

10. The articulation link of claim 1, wherein the body (12) includes at least one lumen (32) extending between the cavity contact surface (20) of the articulation link (10) and the second surface (16).

11. The articulation link of claim 10, wherein the cavity includes a recess (34) configured to receive an elongate member passing through the at least one lumen (32).

12. The articulation link of claim 1, wherein at least one of the cavity sidewall (26) of the articulation link and the protrusion sidewall (28) of the adjacent link includes a contact feature (44) and the other of the cavity sidewall and the protrusion sidewall includes a groove (48) configured to receive a contact feature of the adjacent link.

13. The articulation link of claim 1, wherein the protrusion includes at least two curved rails (42) configured to engage a cavity contact surface (20') of an adjacent link.

14. The articulation link of claim 13, wherein each of the at least two curved rails (42) includes a distal-most convex surface.

15. The articulation link of claim 13, wherein the cavity contact surface (20') of the articulation link (10') includes at least two recesses (46) configured to receive the at least two rails (42) of a second adjacent link.

## Patentansprüche

1. Gelenkverbindung, die aufweist:
einen Körper (12), der eine Längsachse (38) und eine Transversalebene definiert, die sich im Wesentlichen senkrecht zur Längsachse erstreckt, wobei der Körper eine erste Oberfläche (14), die nahe einem ersten Ende (15) des Körpers angeordnet ist, und eine zweite Oberfläche (16) aufweist, die nahe einem zweiten Ende (17) des Körpers gegenüber dem ersten Ende angeordnet ist;
einen Hohlraum (18), der sich von der ersten Oberfläche (14) zu einer Hohlraumkontaktfläche (20) erstreckt, die zwischen der ersten Oberfläche und der zweiten Oberfläche angeordnet ist, wobei sich die Hohlraumkontaktfläche parallel zur Transversalebene erstreckt; und
einen Vorsprung (22), der sich von der zweiten Oberfläche (16) weg von der ersten Oberfläche zu einer Vorsprungkontaktfläche (24) erstreckt, wobei die Vorsprungkontaktfläche gekrümmt und konfiguriert ist, mit einer Hohlraumkontaktfläche einer benachbarten Verbindung in Eingriff zu treten,
wobei der Hohlraum eine Hohlraumseitenwand (26) aufweist, die sich senkrecht zur Transversalebene erstreckt, und der Vorsprung (22) eine Vorsprungseitenwand (28) aufweist, die konfiguriert ist, mit der Hohlraumseitenwand (26) der benachbarten Verbindung in Eingriff zu treten, und
wobei der Vorsprung (22) auf der Längsachse zentriert ist.

2. Gelenkverbindung nach Anspruch 1, wobei die Hohlraumseitenwand (26) der Gelenkverbindung (10) parallel zur Vorsprungseitenwand (28) verläuft.

3. Gelenkverbindung nach Anspruch 2, wobei die Hohlraumseitenwand (26) der Gelenkverbindung (10) und die Vorsprungseitenwand (28) der benachbarten Verbindung flach sind.

4. Gelenkverbindung nach Anspruch 3, wobei die erste Oberfläche und/oder die zweite Oberfläche (14, 16) flach und parallel zur Transversalebene ist.

5. Gelenkverbindung nach Anspruch 2, die ferner mindestens zwei parallele Hohlraumseitenwände (26) und mindestens zwei parallele Vorsprungseitenwände (28) aufweist.

6. Gelenkverbindung nach Anspruch 5, wobei jeder Transversalquerschnitt des Hohlraums und des Vorsprungs rechteckig ist.

7. Gelenkverbindung nach Anspruch 1, wobei die Vorsprungkontaktfläche (24) halbzylindrisch ist.

8. Gelenkverbindung nach Anspruch 1, wobei die Vorsprungkontaktfläche (24) in eine erste Richtung in der Transversalebene konvex ist und in eine zweite Richtung in der Transversalebene konkav ist, wobei die erste Richtung senkrecht zur zweiten Richtung ist.

9. Gelenkverbindung nach Anspruch 1, wobei der Körper (12) ein zentrales Lumen (30) aufweist, das sich longitudinal von der Hohlraumkontaktfläche (20) der Gelenkverbindung zur Vorsprungkontaktfläche (24) erstreckt, und der Körper ferner mindestens ein Lumen (32) aufweist, das sich zwischen der ersten Oberfläche (14) und der zweiten Oberfläche (16) erstreckt.

10. Gelenkverbindung nach Anspruch 1, wobei der Körper (12) mindestens ein Lumen (32) aufweist, das sich zwischen der Hohlraumkontaktfläche (20) der Gelenkverbindung (10) und der zweiten Oberfläche (16) erstreckt.

11. Gelenkverbindung nach Anspruch 10, wobei der Hohlraum eine Aussparung (34) aufweist, die konfiguriert ist, ein längliches Element aufzunehmen, das durch das mindestens eine Lumen (32) geht.

12. Gelenkverbindung nach Anspruch 1, wobei mindestens eine der Hohlraumseitenwand (26) der Gelenkverbindung und der Vorsprungseitenwand (28) der benachbarten Verbindung ein Kontaktmerkmal (44) aufweist und die andere der Hohlraumseitenwand und der Vorsprungseitenwand eine Nut (48) aufweist, die konfiguriert ist, ein Kontaktmerkmal der benachbarten Verbindung aufzunehmen.

13. Gelenkverbindung nach Anspruch 1, wobei der Vorsprung mindestens zwei gekrümmte Schienen (42) aufweist, die konfiguriert sind, mit einer Hohlraumkontaktfläche (20') einer benachbarten Verbindung in Eingriff zu treten.

14. Gelenkverbindung nach Anspruch 13, wobei jede der mindestens zwei gekrümmten Schienen (42) eine am weitesten distal gelegene konvexe Oberfläche aufweist.

15. Gelenkverbindung nach Anspruch 13, wobei die Hohlraumkontaktfläche (20') der Gelenkverbindung (10') mindestens zwei Aussparungen (46) aufweist, die konfiguriert sind, die mindestens zwei Schienen (42) einer zweiten benachbarten Verbindung aufzunehmen.

## Revendications

1. Joint articulé, comprenant :
un corps (12) définissant un axe longitudinal (38) et un plan transversal s'étendant sensiblement perpendiculairement à l'axe longitudinal, ledit corps présentant une première surface (14) proche d'une première extrémité (15) du corps et une deuxième surface (16) proche d'une deuxième extrémité (17) du corps opposée à la première extrémité ;
une cavité (18) s'étendant de la première surface (14) à une surface de contact (20) de cavité située entre la première surface et la deuxième surface, la surface de contact de cavité s'étendant parallèlement au plan transversal ; et
une saillie (22) s'étendant de la deuxième surface (16) à l'opposé de la première surface vers une surface de contact (24) de saillie, la surface de contact de saillie étant courbe et prévue pour s'enclencher avec une surface de contact de cavité d'un joint adjacent,
la cavité comportant une paroi latérale (26) de cavité s'étendant perpendiculairement au plan transversal, et la saillie (22) comportant une paroi latérale (28) de saillie prévue pour s'enclencher avec la paroi latérale (26) de cavité du joint adjacent, et
la saillie (22) étant centrée sur l'axe longitudinal.

2. Joint articulé selon la revendication 1, où la paroi latérale (26) de cavité de joint articulé (10) est parallèle à la paroi latérale (28) de saillie.

3. Joint articulé selon la revendication 2, où la paroi latérale (26) de cavité de joint articulé (10) et la paroi latérale (28) de saillie du joint adjacent sont planes.

4. Joint articulé selon la revendication 3, où la première surface et/ou la deuxième surface (14, 16) sont planes et parallèles au plan transversal.

5. Joint articulé selon la revendication 2, comprenant au moins deux parois latérales (26) de cavité parallèles et au moins deux parois latérales (28) de saillie parallèles.

6. Joint articulé selon la revendication 5, où la section transversale de la cavité et celle de la saillie sont rectangulaires.

7. Joint articulé selon la revendication 1, où la surface de contact (24) de saillie est semi-cylindrique.

8. Joint articulé selon la revendication 1, où la surface de contact (24) de saillie est convexe dans une première direction dans le plan transversal et concave dans une deuxième direction dans le plan transversal, la première direction étant perpendiculaire à la deuxième direction.

9. Joint articulé selon la revendication 1, où le corps (12) comprend une lumière centrale (30) s'étendant longitudinalement de la surface de contact (20) de cavité de joint articulé à la surface de contact (24) de saillie, et où le corps comprend en outre au moins une lumière (32) s'étendant entre la première surface (14) et la deuxième surface (16).

10. Joint articulé selon la revendication 1, où le corps (12) comprend au moins une lumière (32) s'étendant entre la surface de contact (20) de cavité de joint articulé (10) et la deuxième surface (16).

11. Joint articulé selon la revendication 10, où la cavité comporte un évidement (34) prévu pour recevoir un élément allongé passant dans ladite au moins une lumière (32).

12. Joint articulé selon la revendication 1, où la paroi latérale (26) de cavité de joint articulé et/ou la paroi latérale (28) de saillie du joint adjacent présentent un élément de contact (44), et où l'autre paroi, entre la paroi latérale de cavité et la paroi latérale de saillie, présente une rainure (48) prévue pour recevoir un élément de contact du joint adjacent.

13. Joint articulé selon la revendication 1, où la saillie comporte au moins deux rails courbes (42) prévus pour s'enclencher avec une surface de contact de cavité (20') d'un joint adjacent.

14. Joint articulé selon la revendication 13, où chaque rail des au moins deux rails courbes (42) présente une surface distalement convexe.

15. Joint articulé selon la revendication 13, où la surface de contact de cavité (20') de joint articulé (10') présente au moins deux évidements (46) prévus pour recevoir les au moins deux rails (42) d'un deuxième joint adjacent.
